Europäisches Patentamt

European Patent Office

Office européen des brevets

(19)

(11) Veröffentlichungsnummer: **0 268 581 B1**

(12) **EUROPÄISCHE PATENTSCHRIFT**

(45) Veröffentlichungstag der Patentschrift: **08.04.92**

(21) Anmeldenummer: **86903257.3**

(22) Anmeldetag: **20.06.86**

(86) Internationale Anmeldenummer:
**PCT/DE86/00260**

(87) Internationale Veröffentlichungsnummer:
**WO 87/00434 (29.01.87 87/03)**

(51) Int. Cl.⁵: **A61K 31/557**, //(A61K31/557, 31:18)

(54) **PROSTACYCLINE, IHRE ANALOGE ODER PROSTAGLANDINE UND THROMBOXANANTAGONISTEN ZUR BEHANDLUNG VON THROMBOTISCHEN UND THROMBOEMBOLISCHEN KRANKHEITSBILDERN.**

(30) Priorität: **19.07.85 DE 3526362**

(43) Veröffentlichungstag der Anmeldung:
**01.06.88 Patentblatt 88/22**

(45) Bekanntmachung des Hinweises auf die Patenterteilung:
**08.04.92 Patentblatt 92/15**

(84) Benannte Vertragsstaaten:
**AT BE CH DE FR GB IT LI LU NL SE**

(56) Entgegenhaltungen:
**CH-A- 637 298**

(73) Patentinhaber: **SCHERING AKTIENGESELL-SCHAFT Berlin und Bergkamen Müllerstrasse 170/178 Postfach 65 03 11 W-1000 Berlin 65(DE)**

(72) Erfinder: **STÜRZEBECHER, Claus-Steffen Brigittenstrasse 6a W-1000 Berlin 46(DE)**
Erfinder: **WITT, Werner, Georg Motzstrasse 25 W-1000 Berlin 30(DE)**
Erfinder: **RADÜCHEL, Bernd Gollanzstrasse 132 W-1000 Berlin 28(DE)**
Erfinder: **SKUBALLA, Werner Ohvenstrasse 23 W-1000 Berlin 28(DE)**
Erfinder: **VORBRÜGGEN, Helmut Wilkestrasse 7 W-1000 Berlin 27(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 268 581 B1

**Beschreibung**

Die Erfindung betrifft ein Kombinationserzeugnis zur gemeinsamen Verwendung der Behandlung thrombotischer und thromboembolischer Krankheitsbilder mit Zuständen erhöhter intravasaler Plättchenaktivierung und damit erhöhter Plättchenaggregations- und Plättchenadhäsionsneigung sowie erhöhter Blutgerinnungsneigung, enthaltend ein Prostacyclin, ein Prostacyclinanalogon oder ein Prostaglandin (PC/PCA/PG) und einen Thromboxanrezeptorantagonisten (TXAA).

Die Erfindung betrifft auch die Verwendung von PC/PCA/PG in Kombination mit einem Thromboxanrezeptorantagonisten zur Anwendung bei: Prophylaxe und Therapie koronarer Herzkrankheiten, koronarer Thrombose, Herzinfarkt, peripherer Arterienerkrankung, diabetischen Gefäßveränderungen, Arteriosklerose und Thrombose, Schlaganfall, Prophylaxe und Therapie ischaemischer Attacken des ZNS-Systems, Migräne, Therapie des Schocks, Inhibierung der Bronchokonstriktion, Inhibierung der Magensäuresekretion; weitere Verwendungsmöglichkeiten der Kombination sind Zytoprotektion der Magen- und Darmschleimhaut, Zytoprotektion in der Leber, Niere und im Pankreas, Senkung des pulmonaren vaskulären Widerstandes und des pulmonaren Blutdruckes, Förderung der Nierendurchblutung, Verwendung für die Herstellung von Arzneimitteln zur Anwendung anstelle von Heparin oder zur Anwendung als Adjuvans bei der Dialyse, der Hämofiltration, Konservierung von Blutplasmakonserven, besonders von Blutplättchenkonserven, Inhibierung von Geburtswehen, Behandlung von Schwangerschaftstoxikosen, Erhöhung der zerebralen Durchblutung etc.; zusätzlich kann das Kombinationserzeugnis als Antiallergikum und als antiproliferative Wirksubstanz eingesetzt werden.

Die Kombination kann zum Beispiel auch gemeinsam mit Calciumantagonisten, Thromboxansynthetasehemmstoffen, typischen Antikoagulantien wie Heparin, Cumarin oder Aspirin und entsprechenden Wirkstoffen, mit Phosphodiesterasehemmern, mit Angiotensin-Converting-Enzyme (ACE)-Inhibitoren, mit vasodilatierenden Wirkstoffen insbesondere mit $\beta$-Rezeptor-Blockern, Antiphlogistika, Antipyretika und Antiallergika etc. angewendet werden.

Es ist bereits bekannt, daß zur Behandlung akuter und chronischer thrombotischer und thromboembolischer Erkrankungen Prostaglandin $E_1$, Prostacyclin und Prostacyclinanaloga verwendet werden [Gebrauchsinformation für Prostavasin $^{(R)}$ der Fa. Sanol Schwarz GmbH; Moncada. S., Br. J. Pharmacol. 76/1, 3-31 (1982); Schillinger, E.,T. Krais, G. Stock, New Drug Annual: Cardivascular Drugs, Raven Press, im Druck].

Neben den Vorteilen dieser Behandlungsformen, die in einer starken Hemmung aller Stimulatoren der Plättchenaggregation bestehen, sind diese Therapien durch die kardiovaskulären Nebenwirkungen, insbesondere die ausgeprägte Blutdrucksenkung, begrenzt.

Auch die in CH-A-637298 beschriebene Kombination von aggregationshemmender Substanz, Antioxydans und/oder Thromboxan $A_2$-Synthetase-Inhibitor verursacht eine so starke Blutdrucksenkung, daß sie sogar als antihypertensives Mittel zur Behandlung von hohem Blutdruck bezeichnet wird.

Für TXAA ist in den bisherigen klinischen Untersuchungen kein ausreichender Wirknachweis für dieses Therapieprinzip geführt worden.

Über eine Plasmaspiegelerhöhung des endogenen Prostacyclins in Kombination mit einem TXAA zu besseren antiaggregatorischen wirksamen Prinzipien zu kommen [Chan, C-C.,A.Ford-Hutchinson, Europ.J.of Pharmacol. 110,323-328, (1985)] ist als unzureichend zu betrachten, insbesondere da eine Dosierbarkeit und überhaupt ein sicherer und reproduzierbarer Anstieg des endogenen Prostacyclins nicht möglich ist. Darüber hinaus besteht beim endogenen Prostacyclin nicht die Möglichkeit, wie bei Anwendung synthetischer PC/PCA/PG, erwünschte Wirkungen von Nebenwirkungen des Prostacyclins zu trennen.

Es wurde nun überraschenderweise gefunden, daß die für PC/PCA/PG typischen Nebenwirkungen und die nicht ausreichende Wirksamkeit von TXAA vermieden bzw. ausgeglichen werden, wenn man PC/PCA/PG und TXAA gemeinsam bei der Behandlung der oben genannten Krankheitsbilder verwendet. Während die plättchenhemmende antithrombotische und antithrombogenetische Wirkung beider Wirkstoffklassen gegenseitig potenziert wird, kommt es zu einer Verminderung der kardiovaskulären Nebenwirkungen des PC/PCA/PG infolge der bei Kombination möglichen Mengenreduktion der einzelnen Wirkstoffe. Dadurch wird die therapeutische Breite der einzelnen Wirkstoffe vergrößert.

Es können Gewichtsmengen an Prostacyclin/Prostacyclinanalogon/Prostaglandin und an Thromboxanrezeptorantagonist verwendet werden, die bei gemeinsamer Anwendung im Vergleich zu den bisher verwendeten erforderlichen Dosierungen der einzelnen Wirkstoffe stark herabgesetzt sind.

PC/PCA/PG und TXAA werden kombiniert in einer Dosiseinheit oder getrennt und gleichzeitig oder sequentiell in einem Gewichtsverhältnis von im wesentlichen ca. 1 : 1 bis 1 : 10 000 verwendet (z.B. im gleichen Vehikel, einer Tablette oder auch einer öligen Lösung wie ein Benzylbenzoat/Rizinus-Gemisch).

Der sequentiellen Behandlung kommt insofern besondere Bedeutung zu, als bei solchen Behandlungen,

2

bei denen eine Abnahme der PC/PCA/PG Wirkung auftritt oder zu erwarten ist (Tachyphylaxie, [Sinzinger, H., S. Reiter, Prostagl. Leukotr. and Medicine 13/3, 281-288 (1984)]), durch wechselseitige ein- und ausschleichende Therapie mit PC/PCA/PG und TXAA eine solche Wirkungsverminderung verhindert werden kann.

Als für die erfindungsgemäße Verwendung geeignete Verbindungen der Prostacyclin-/Prostacyclinanalogon-/Prostaglandin-Reihe kommen alle Substanzen in frage, die am Blutplättchen aggregationshemmende Eigenschaften aufweisen und beispielsweise beschrieben sind in:

R.C. Nickolson, M.H. Town und H. Vorbrüggen, Med.Res.Rev. 5 (1985), B.I.R. Whittle und S. Moncada, Progress in Medicinal Chemistry 21, 236 (1984), P.A. Aristoff in Advances in Prostaglandin, Thromboxane and Leukotriene Research Vol. 14, 1985, p.309, B. Radüchel und H. Vorbrüggen in Advances in Prostaglandin, Thromboxane and Leukotriene Research Vol. 14, 1985, p.263,

in den Europäischen Patentanmeldungen der Veröffentlichungsnummern 0011591, 0055208, 0069692, 0086404, 0099538, 0119949 sowie in den DE-OS 34 08 699 und DE-OS 35 10 978.

Beispielsweise genannt seien:

Prostacyclin $PGI_2$,

15-Cyclopentyl-$\omega$-pentanor-5(E)-carbacylic (ONO-41 483; Prost. and Med. 10, 53, (1983),

5-{(E)-(1S,5S,7R)-7-Hydroxy-6-[(3S,4S)-3-hydroxy-4-methyl-nona-1,6-diinyl]-bicyclo[3.3.0]-octan-3-yliden}-pentansäure (EP 0086404),

(5E)-(16S)-13,14-Didehydro-16,20-dimethyl-18,18,19,19-tetradehydro-2,3,4-trinor-1,5     inter-m-phenylen-6a-carba-prostaglandin $I_2$ (DE-OS 3408699),

5-{(E)-(1S,5S,6R,7R)-7-Hydroxy-6-[(3S,4RS)-3-hydroxy-4-methyl-1-octen-6-inyl]-bicyclo[3.3.0]-octan-3-yliden}-pentansäure (Iloprost, EP 0011591),

7-{(E)-(1S,5S,6S,7R)-7-Hydroxy-6-[(3S,4S)-3-hydroxy-4-methyl-nona-1,6-diinyl]bicyclo[3.3.0]-octan-3-yliden}-5-oxa-heptansäure (EP 0099538),

(5Z,13E,9$\alpha$,11$\alpha$,15S)-2,3,4-Trinor-inter-m-phenylen-6,9-epoxy-11,15-dihydroxy-15-cyclohexyl-16,17,18,19,20-pentanor]-prosta-diensäure (C G 4305, Arzn, Forsch. 1983, 1240) sowie das entsprechende Natriumsalz (C G 4203, Drugs Future 9, 494, (1984)),

$\beta$-Thia-imino-Prostacyclin (Hoe 892, Prost. and Med. 10, 231 (1983)), 9-Methyl-carbacyclin (J. Org.Chem. 48, 534 (1983)), 5-{(E)-(1S,5S,6S,7R)-7-Hydroxy-6-[(3S,4S)-3-hydroxy-4-methyl-nona-1,6-diinyl]-bicyclo-[3.3.0]-octan-3-yliden}-3-oxa-pentansäure (EP 0119949),

5-{(Z)-(1S,5S,6S,7R)-7-Hydroxy-6-[(3S,4S]-3-hydroxy-4-methyl-nona-1,6-diinyl]-bicyclo[3.3.0]-octan-3-yliden}-3-oxa-5-fluor-pentansäure (EP 0099538),

7-Oxo-16-methyl-18,19-didehydro-$PGI_2$ (DE-OS 3 035 454), 7-Oxo-$PGI_2$ (DE-OS 3 035 454),

Prostaglandin $E_1$,

6-Keto-Prostaglandin $E_1$,

(5Z,13E)-(9R,11R,15S)-9-Chlor-15-cyclohexyl-11,15-dihydroxy-16,17,18,19,20-pentanor-5,13-prostadiensäure (DE-OS 3 510 978),

17S,20-Dimethyl-trans-2,3-didehydro-$PGE_1$ (ONO 1206, Drugs Future 7, 116 (1982)).

Anstelle der angegebenen Prostan-säuren können auch deren physiologisch verträgliche Salze mit anorganischen oder organischen Basen, wie zum Beispiel Natriumhydroxid, Kaliumhydroxid, Tris-(hydroxymethyl)-aminomethan, Glucamin, N-Methylglucamin, Morpholin, Lysin, Arginin verwendet werden.

Als Thromboxanrezeptorantagonisten kommen alle Verbindungen in Frage, die eine ausreichende Affinität zum Thromboxanrezeptor besizen und keine oder im verwendeten Dosisbereich nur unbedeutende thromboxanagonistische Aktivität besitzen, wie sie z.B. beschrieben sind in: J5 5017-315; US 4472-586-A; US 4263-207; US 4394-515-A; US 4282-365; BE-883-713; J5 7093-962; J6 0004-154-A; EP--43-292; EP--82-646-A; DE 3346-047-A; WO 8400-754-A; US 4474-804-A; DE 3401-986-A; DE 3127-343; BE-897-763-A; EP--74-861; AU 8425-607-A; EP--78-668; DE 3339-019-A; EP-137-426-A und bei N.H. Wilson und R.L. Jones in: Advances in Prostaglandin, Thromboxane and Leukotriene Research 14, 420-423 (1985), sowie von K. Stegmeier et al. in: Thrombosis Research 35, 379-395 (1984).

Beispielsweise genannt seien:

4-[2-(Benzolsulfonamido)-ethyl]-phenoxyessigsäure (BM 13 177) [K. Stegmeier et al. in: Thrombosis Research 35, 379-395, 1984].

[1$\alpha$(Z),2$\beta$,5$\alpha$]-($\pm$)-7-[5-[[(1,1-biphenyl)-4-yl]methoxy]-2-(4-morpholinyl)-3-oxocyclopentyl]-4-hepatonoic     acid (AH 23848) (Br. J. Pharmac. (1985), 86,259)

[1$\beta$,2$\alpha$(5Z),3$\alpha$,4$\beta$]-7-[[2-[(phenylamino)carbonyl]hydrazino]methyl]-7-oxabicyclo[2.2.1] hept-2-yl]-5-heptenoic acid (SQ 29.548) (Prostaglandins 29, 785 (1985))

4-(4-Chlorbenzolsulphonylamino)-ethyl-benzol-essigsäure (BM 13 505) (Intern. Conf.Leukotrienes and Prostanoids in Health and Disease, Tel-Aviv , Oct. 1985, p. 10)

7-[3-[[[(phenylamino)carbonyl]hydrazonol]methyl]bicyclo[2.2.1] hept-2-yl]-,[1$\alpha$, 2$\beta$,(Z), 3$\alpha$, 4$\alpha$] (EP 045) und 7-{(1S,2S,3S,4R)-3-[1-(3-phenylthioureidoimino)ethyl]-bicyclo[2.2.1]heptan-2-yl}-5-heptensäure (EP 092) [beide Substanzen R.A. Armstrong et al. in Br.J.Pharmacol. 84, 595-607, 1985].

Dibenzo[b,f]thiepin-3-methanol,5,5-dioxid (L 640035) [C̄-C̄. Chan in: Europ.J.Pharmacol. 110 (3), 323-328. 1985].

2,7(1H)-Isochinolindisulfonamide, N7-(3-chlorophenyl)-N2-[[7-[[(3-chlorophenyl)amino]sulfonyl]-3,4-dihydro-2(1H)-isochinolinal]sulfonyl]-3,4-dihydro (SKF 88046) [8.M. Weichman et al. in: Prostaglandins Leukotrienes and Medicine 15, 167-175, 1984].

Die PC/PCA/PG werden in Mengen eingesetzt, die deutlich unterhalb der sonst zur Hemmung der intravasalen Plättchenaggregation eingesetzten Mengen liegen. Bei der Verwendung von Iloprost als PCA wird man gemäß vorliegender Erfindung in der Regel mit 10 - 1000 $\mu$g, vorzugsweise mit 50 - 250 $\mu$g pro Tag auskommen. Die Applikation kann zum Beispiel enteral oder parenteral, inhalativ oder auch transdermal oder lokal erfolgen.

Bei der intravenösen Infusion von Iloprost werden beispielsweise Mengen von etwa 50 - 150 $\mu$g Pro Tag benötigt. Bei der oralen Applikation werden etwa 125 - 250 $\mu$g pro Tag eingesetzt.

Eine Dosiseinheit von Iloprost enthält ca. 100 $\mu$g/ml als Stammlösung zur Verdünnung in üblichen Infusionsträgerlösungen für die intravenöse Anwendung,

Für die orale Anwendung enthält eine Dosiseinheit 25 - 50 $\mu$g, bei Retardformulierungen 25 - 250 $\mu$g, als Tablette, Dragee, Kapsel, Pille, Suspension oder Lösung, die in üblicher Weise mit den in der Galenik gebräuchlichen Zusätzen und Trägersubstanzen hergestellt werden können. Für die lokale, topische oder transdermale Anwendung kommen beispielsweise Systeme wie Hautpflaster oder Suppositorien in Frage.

Erfindungsgemäß können auch biologisch äquivalente Mengen von anderen Prostacyclinanaloga oder Prostaglandinen eingesetzt werden.

Die Thromboxanrezeptorantagonisten werden gemäß vorliegender Erfindung in Mengen eingesetzt, die in der Regel unterhalb der bisher in Humanstudien verwendeten Mengen liegen [Riess, H., E. Hiller, B. Reinhardt, C. Bräuning in:

Thrombosis Research 35, 371-378, 1984]. Im allgemeinen reichen 100 - 2 000 mg/Tag, vorzugsweise 200 - 800 mg/Tag BM 13 177. bzw. 1-100 mg/Tag, vorzugsweise 2-50 mg/Tag, AH23848 oder SQ 29548, oder eine biologisch äquivalente Menge eines anderen Thromboxanrezeptorantagonisten.

Die TXAA können zum Beispiel enteral oder parenteral, inhalativ aber auch transdermal oder lokal appliziert werden.

Für die bevorzugte orale Applikation kommenen insbesondere Tabletten, Dragees, Kapseln, Pillen, Suspensionen oder Lösungen in Frage, die in üblicher Weise mit den in der Galenik gebräuchlichen Zusätzen und Trägersubstanzen hergestellt werden können.

Für die lokale Anwendung können beispielsweise transdermale Systeme wie Hautpflaster verwendet werden.

Eine Dosiseinheit für die orale oder parenterale Anwendung enthält etwa 50 - 300 mg, als Retardformulierung 100 - 1000 mg, BM 13 177/Tag oder eine biologisch äquivalente Menge eines anderen Thromboxanrezeptorantagonisten.

Die gemeinsame Behandlung mit PCA und TXAA erfolgt, je nachdem ob es sich um ein akutes, subakutes oder ein chronisches Krankheitsgeschehen handelt, über einige Tage bis zu Wochen und Monaten, wobei PCA und TXAA in einer Dosiseinheit oder getrennt und gleichzeitig oder sequentiell verabreicht werden.

Die nachstehenden Beispiele sollen die galenische Formulierung erläutern:

Beispiel 1 Zusammensetzung einer Kombinationstablette

|  | % | Menge/Tablette [mg] |
|---|---|---|
| 1. Iloprost (als Lsg. in Ethanol 50%) | 0,01 | 0,05 |
| 2. BM 13 177 | 50,0 | 250,0 |
| 3. Laktose | 34,99 | 174,95 |
| 4. Maisstärke | 10,0 | 50,0 |
| 5. Polyvinylpyrrolidon 2500 | 3,0 | 15,0 |
| 6. Stearinsäure | 2,0 | 10,0 |
|  | 100 % | 500,00 mg |

Die Bestandteile 3, 4 und 5 werden gesiebt, gemischt und mit der Lösung von 1 granuliert. Nach dem Trocknen werden 2 und 6 nacheinander untermischt und die Preßmasse zu runden Tabletten mit 11 mm Durchmesser verpreßt.

Beispiel 2 Zusammensetzung einer Kombinationstablette

|  | % | Menge/Tablette [mg] |
|---|---|---|
| 1. Iloprost (als Lsg. in Ethanol 50%) | 0,02 | 0,05 |
| 2. SQ 29 458 | 4,0 | 10,0 |
| 3. Laktose | 65,98 | 164,95 |
| 4. Maisstärke | 20,0 | 50,0 |
| 5. Polyvinylpyrrolidon 2500 | 6,0 | 15,0 |
| 6. Stearinsäure | 4,0 | 10,0 |
|  | 100 % | 25.0,00 mg |

Die Bestandteile 3, 4 und 5 werden gesiebt, gemischt und mit der Lösung von 1 granuliert. Nach dem Trocknen werden 2 und 6 nacheinander untermischt und die Preßmasse zu runden Tabletten mit 11 mm Durchmesser verpreßt.

Beispiel 3 Zusammensetzung einer Kombinationstablette

|  | % | Menge/Tablette [mg] |
|---|---|---|
| 1. Iloprost (als Lsg. in Ethanol 50%) | 0,02 | 0,05 |
| 2. AH 23 848 | 1,0 | 2,5 |
| 3. Laktose | 68,98 | 172,45 |
| 4. Maisstärke | 20,0 | 50,0 |
| 5. Polyvinylpyrrolidon 2500 | 6,0 | 15,0 |
| 6. Stearinsäure | 4,0 | 10,0 |
|  | 100 % | 250,00 mg |

Die Bestandteile 3, 4 und 5 werden gesiebt, gemischt und mit der Lösung von 1 granuliert. Nach dem Trocknen werden 2 und 6 nacheinander untermischt und die Preßmasse zu runden Tabletten mit 11 mm Durchmesser verpreßt.

Beispiel 4 Zusammensetzung einer Kombinationstablette

|  | % | Menge/Tablette [mg] |
|---|---|---|
| 1. Iloprost-Na-Salz (gelöst in Aqua dest) | 0,0104 | 0,052 (≙ 0,05 mg Iloprost) |
| 2. BM 13 177 | 50,0 | 250,0 |
| 3. Laktose | 34,99 | 174,95 |
| 4. Maisstärke | 10,0 | 50,0 |
| 5. Polyvinylpyrrolidon 2500 | 3,0 | 15,0 |
| 6. Stearinsäure | 2,0 | 10,0 |
|  | 100 % | 500,0 mg |

Die Bestandteile 3, 4 und 5 werden gesiebt, gemischt und mit der Lösung von 1 granuliert. Nach dem Trocknen werden 2 und 6 nacheinander untermischt und die Preßmasse zu runden Tabletten mit 11 mm Durchmesser verpreßt.

Beispiel 5 Zusammensetzung einer Kombinationstablette

6

| | % | Menge/Tablette [mg] |
|---|---|---|
| 1. Iloprost als Einschlußverbindung mit α, ß oder ϒ-Cyclodextrin, 3 % (g/g) | 0,33 | 1,66 (≙ 0,05 mg Iloprost) |
| 2. BM 13 177 | 50,0 | 250,0 |
| 3. Mikrokristalline Cellulose | 35,67 | 178,34 |
| 4. Maisstärke | 12,0 | 60,0 |
| 5. Stearinsäure | 2,0 | 10,0 |
| | 100 % | 500,0 mg |

Die Rezepturbestandteile werden mit Ausnahme der Stearinsäure gesiebt und 15 Minuten gemischt. Stearinsäure (gesiebt) wird hinzugefügt und weitere 3 Minuten mit den übrigen Rezepturbestandteilen gemischt. Die Preßmasse wird zu runden Tabletten mit 11 mm Durchmesser verpreßt.

PHARMAKOLOGISCHE BEOBACHTUNGEN

1. Thrombozytenfunktion in vitro (Human-PRP).

Es werden BM 13 177 (im Text BM) und Iloprost in ihrer Wirkung auf die Thrombozytenaggregation und den Thrombozytenformwandel (shape change) gegen die Stimulatoren U 46 619 (9,11-dideoxy, $9\alpha$-$11\alpha$-methanoepoxy-PGF$_{2\alpha}$ , stabiler TXA$_2$-Agnoist) und ADP geprüft und die IC$_{50}$-Werte ermittelt. Danach werden in mehreren unabhängigen Versuchen die Wirkung einer Kombination beider Substanzen auf die Aggregation (U 46 619 und ADP) in verschiedenen Konzentrationen geprüft; insbesondere wird die Kombination niedriger Dosierungen und im Bereich der IC$_{50}$-Werte liegender Dosierungen beider Substanzen geprüft.

Methodik

Plättchenaggregation und Shape change werden als lichtoptische Phänomene in stimulierten PRP-Proben (Platelet rich plasma) gemessen. Dabei wird der Formwandel als Dichtezunahme der Probe durch Übergang der diskoiden Ruheform des Plättchens in eine sphärozytäre Form mit Ausbildung von pseudopodienartigen Membranausbildungen erfaßt und auf einem Schreiber dargestellt. Die Aggregation wird als Dichteabnahme durch Verklumpung und "Ausfällen" von Plättchenaggregaten photometrisch erfaßt und auf einem Schreiber dargestellt.

Ergebnisse

Iloprost hemmt konzentrationsabhängig die durch U 46 619 induzierte Plättchenaggregation und den Formwandel. Die IC$_{50}$ für die Aggregationshemmung ist 1,3 - 2,6 nM, für die Shape-change-hemmung beträgt die IC$_{50}$ 0,52 - 1,3 nM.

Die 2. Welle der durch ADP induzierten Aggregation wird mit einer IC$_{50}$ von 0,26 - 0,65 nM konzentrationsabhängig gehemmt.

BM 13 177 hemmt konzentrationsabhängig die durch U 46 619 induzierte Plättchenaggregation und den Formwandel. Die IC$_{50}$ beträgt für die Aggregation 1,65 - 6,6 $\mu$M, für den Shape change 1,65 - 3,3 $\mu$M.

Die 2. Welle der ADR induzierten Aggregation wird mit einer IC$_{50}$ von 0,33 - 0,66 $\mu$M konzentrationsabhängig gehemmt.

Kombination von Thromboxanrezeptorantagonist und Iloprost

Bei der Kombination von BM 13 177 mit Iloprost werden die durch U 46 619 induzierte Plättchenaggregation und der Plättchenformwandel und die 2. Welle der durch AdP induzierten Aggregation bei Konzentrationen beider Wirkstoffe, die im Schwellen- oder Unterschwellenbereich liegen, stark gehemmt. Die Hemmeffekte beider Wirkstoffe potenzieren sich dabei (siehe Tabelle).

## Tabellen

### Beispiel 1: Aggregation durch 100 ng/ml U 46 619.

| Wirkstoff | Konzentration | % Hemmung |
|---|---|---|
| 1 BM 13 177 | 0,66 μM | 16 % |
| 2 Iloprost | 0,1 ng/ml | 2 % |
| 1 + 2 | | 65 % |

### Beispiel 2: Shape change durch 50 ng/ml U 46 619.

| Wirkstoff | Konzentration | % Hemmung |
|---|---|---|
| 1 BM 13 177 | 0,66 μM | 28 % |
| 2 Iloprost | 0,1 ng/ml | keine Hemmung |
| 1 + 2 | | 61 % |

### Beispiel 3: 2. Welle der Aggregation durch $0,5 \times 10^{-6}$ M ADP.

| Wirkstoff | Konzentration | % Hemmung |
|---|---|---|
| 1 BM 13 177 | 0,165 μM | keine Hemmung |
| 2 Iloprost | 0,1 ng/ml | keine Hemmung |
| 1 + 2 | | 60 % |

2. Intravasale Thrombozytenaggregation an der narkotisierten Ratte.

Methodik

Die Beeinflussung der intravasalen Thrombozytenaggregation wird an Urethan-narkotisierten Ratten untersucht.

Kollagen (100 μg/kg i.v. Bolus) erzeugt transitorische Thrombozytopenien (einen vorübergehenden Abfall der Blutplättchenkonzentration), gemessen durch kontinuierliche Blutentnahme aus der A. carotis (50 μl/min) und Thrombozytenzählung im Technicon-Autocounter. Als Maß für die Hemmung der intravasalen Plättchenaggregation dient die Änderung der Kollagen-induzierten Thrombozytopenie (% Abfall) im Vergleich zum Ausgangswert ( = 2. Kollageninjektion).

Ergebnisse

Iloprost (0,1-0,33-1,0 $\mu$g/kg/min) hemmt die Kollagen-induzierte Thrombozytopenie dosisabhängig. BM 13 177 hemmt die Thrombozytopenie in der niedrigsten Dosierung (0,5 mg/kg/min) um 27 ± 6 % (MW ± SE), ein Effekt der durch Dosiserhöhung (1,0-2,0 mg/kg/min) nicht steigerbar ist. Die Kombination einer Schwellendosis von Iloprost (0,1 $\mu$g/kg/min) mit der niedrigsten getesteten Dosis von BM 13 177 (0,5 mg/kg/min) ergibt eine hinsichtlich der Einzelgaben signifikant ($\alpha$ = 0.05; Lord-Test) stärkere Inhibition von 45 ± 2 %.

3. Mesenterialarteriolenthrombose an narkotisierten Meerschweinchen.

Methodik

An narkotisierten Meerschweinchen wird eine Mesenterialschlinge zur Vitalmikroskopie präpariert. Nach elektrischer Läsion einer Arteriole wird unter Kontrollbedingungen und nach Substanzapplikation ADP bis zum Auffinden einer thrombogenen Dosis (Okklusion durch Plättchenthrombus) topisch appliziert.
Parameter: Erhöhung der thrombogenen ADP-Konzentration vs. Vorwert; i.v.-Infusion von Iloprost, i.v. Injektion von BM 13 177.

Ergebnisse

An elektrisch vorgeschädigten Mesenterialarteriolen narkotisierter Meerschweinchen erhöht BM 13 177 (25 mg/kg i.v.) die zur Auslösung okkludierender Plättchenthromben notwendige ADP-Konzentration signifikant um etwa den Faktor 4. Iloprast steigert in der Schwellendosis von 0,1 $\mu$g/kg/min i.v. die thrombogene ADP-Konzentration um den Faktor 2,8.
Die Kombination von BM 13 177 (25 mg/kg i.v.) gefolgt von einer i.v.-Infusion von Iloprost (0,1 $\mu$g/kg/min) erhöht die thrombogene ADP-Konzentration um den Faktor 12.
An einem Versuchsmodell für die arterielle plättcheninduzierte Thrombose führt die Kombination des TXA$_2$-Antagonisten BM 13 177 und des Prostacyclinanalogon Iloprost zu einer potenzierten antithrombogenetischen Wirkung.

4. Jugularvenenthrombose an der narkotisierten Ratte.

Methodik

Durch Belastung der Jugularvene Urethan-narkotisierter Ratten mit einem auf -15°C gekühlten Metallstempel (200 g, 2 min) werden die Gefäße vorgeschädigt. Der innerhalb von 3 Stunden an dieser Stelle gewachsene Thrombus wird durch Bestimmungen seines Hb-Gehaltes (Differenz geschädigtes - ungeschädigtes Gefäßsegment) quantifiziert (Hb-Gehalt Feuchtgewicht).
Die Infusion der Testsubstanzen beginnt 15 min vor Schädigung der Vene und wird bis zum Versuchsende fortgesetzt.

Ergebnisse

Die Infusion von Iloprost, in einer als Einzelgabe nicht signifikant wirksamen Dosierung (30 ng/kg/min, i.v.) in Kombination mit einer ebenfalls als Monotherapie unwirksamen Dosis BM 13 177 (20 mg/kg, i.v. Bolus + 50 $\mu$g/kg/min, i.v.) ergibt eine signifikante Reduzierung des Hb-Gehaltes der Thromben auf Werte ungeschädigter Kontrollen.
BM 13 177 als Monotherapie ist am Jugularvenenthrombosemodell der Ratte nicht antithrombotisch wirksam. Die Kombination mit einer hämodynamisch und antiaggregatorisch unwirksamen Dosis von Iloprost unterdrückt das Thrombuswachstum vollständig.

5. Blutdruck spontan hypertoner (SH) Ratten.

Methodik

An wachen SH-Ratten, denen ein Venenkatheter zur Substanzapplikation und ein Arterienkatheter zur Blutdruckmessung implantiert wird, wird unter Infusion der Prüfsubstanzen das Verhalten von Blutdruck und Herzfrequenz beobachtet.

Iloprost wird in einer Schwellendosis von 0,3 $\mu$g/kg/min (20% Senkung des diastolischen Blutdruckes) mit einer hohen Dosis von BM 13 177, 2 mg/kg/min (siehe Plättchenhemmung in vivo, 27 % Hemmung bei 2 mg/kg i.v. Infusion) für 20 Minuten gemeinsam infundiert (n = 6 Tiere).

Ergebnis

Die durch Iloprost bewirkten hämodynamischen Veränderungen, Blutdruckabfall und Anstieg der Herzfrequenz, werden durch BM 13 177 nur geringfügig beeinflußt.

Während die maximale Blutdrucksenkung unter Iloprost durch BM 13 177 nicht beeinflußt wird, klingt die Wirkung nach Ende der Infusion unter der Kombinationsbehandlung schneller ab.

Eine Verstärkung der blutdrucksenkenden Wirkung von Iloprost durch BM 13 177 muß auch bei hohen Dosierungen von BM 13 177 nicht erwartet werden.

**Patentansprüche**

1. Kombinationserzeugnis zur gemeinsamen Anwendung bei thrombotischen und thromboembolischen Krankheitsbildern, enthaltend Prostacyclin, ein Prostacyclinanalogon oder ein Prostaglandin (PC/PCA/PG) und einen Thromboxanrezeptorantagonisten (TXAA).

2. Erzeugnis gemäß Anspruch 1, dadurch gekennzeichnet, daß PC/PCA/PG und TXAA in einem Gewichtsverhältnis 1:1 bis 1:10000 stehen.

3. Erzeugnis gemäß Anspruch 1, dadurch gekennzeichnet, daß PC/PCA/PG und TXAA in getrennten Dosiseinheiten vorliegen.

4. Erzeugnis gemäß Anspruch 1, dadurch gekennzeichnet, daß PC/PCA/PG und TXAA zusammen in einer Dosiseinheit vorliegen.

5. Erzeugnis gemäß Anspruch 1, dadurch gekennzeichnet, daß eine PC/PCA/PG-Dosiseinheit 25-250 $\mu$g Iloprost = 5-{(E)-(1S,5S,6R,-7R)-7-Hydroxy-6-[(E)-(3S,4RS)-3-hydroxy-4-methyl-1-octen-6-inyl]-bicyclo-[3.3.0]-octan-3-yliden}-pentansäure oder eine biologisch äquivalente Menge eines anderen PC/PCA/PG enthält.

6. Erzeugnis gemäß Anspruch 1, dadurch gekennzeichnet, daß eine TXAA-Dosiseinheit 50-1000 mg BM 13 177 = 4-[2-(Benzolsulfonamido)-ethyl]-phenoxyessigsäure oder eine biologisch äquivalente Menge eines anderen TXAA enthält.

7. Verwendung von PC/PCA/PG und TXAA für die Herstellung von Arzneimitteln zur gleichzeitigen getrennten oder zeitlich abgestuften Anwendung zur Behandlung von thrombotischen oder thromboembolischen Krankheitsbildern.

8. Verwendung von in einer Dosiseinheit vorliegenden PC/PCA/PG und TXAA für die Herstellung von Arzneimitteln zur Anwendung bei Behandlung von thrombotischen oder thromboembolischen Krankheitsbildern.

9. Verwendung von PC/PCA/PG und TXAA für die Herstellung von Arzneimitteln nach Anspruch 7 oder 8, dadurch gekennzeichnet, daß PC/PCA/PG und TXAA in einem Gewichtsverhältnis 1:1 bis 1:10000 stehen.

10. Verwendung von 25-250 g Iloprost = 5-{(E)-(1S,5S,6R,7R)-7-Hydroxy-6-[(E)-(3S,4RS)-3-hydroxy-4-methyl-1-octen-6-inyl]-bicyclo[3.3.0]-octan-3-yliden}-pentansäure als PC/PCA/PG oder einer biologisch äquivalenten Menge eines anderen PC/PCA/PG für die Herstellung von Arzneimitteln gemäß Anspruch 7 oder 8.

11. Verwendung von 50-1000 mg BM 13 177 = 4-[2-(Benzolsulfonamido)-ethyl]-phenoxyessigsäure als TXAA oder einer biologisch äquivalenten Menge eines anderen TXAA für die Herstellung von Arzneimitteln gemäß Anspruch 7 oder 8.

12. Verwendung von PC/PCA/PG und TXAA für die Herstellung von Arzneimitteln zur sequentiellen Anwendung zur Vermeidung der Wirkungsabschwächung der Plättchenaggregationshemmung bei der Behandlung von thrombotischen und thromboembolischen Krankheitsbildern.

## Claims

1. Combination product for combined administration in thrombotic and thrombo-embolic syndromes, containing prostacyclin, a prostacyclin analogue or a prostaglandin (PC/PCA/PG) and a thromboxane receptor antagonist (TXAA).

2. Product according to claim 1, characterised in that the PC/PCA/PG and TXAA are in a weight ratio of from 1:1 to 1:10,000.

3. Product according to claim 1, characterised in that the PC/PCA,/PG and TXAA are present in separate dose units.

4. Product according to claim 1, characterised in that the PC/PCA/PG and TXAA are present together in one dose unit.

5. Product according to claim 1, characterised in that one PC/PCA/PG dose unit contains 25 - 250 $\mu$g of iloprost = 5-{(E)-(1S,5S,6R,7R)-7-hydroxy-6-[(E)-(3S,4RS)-3-hydroxy-4-methyl-1-octen-6-ynyl]-bicyclo-[3.3.0]octa-3-ylidene)-pentanoic acid or a biologically equivalent amount of another PC/PCA/PG.

6. Product according to claim 1, characterised in that one TXAA dose unit contains 50 - 1000 mg of BM 13 177 = 4-[2-(benzenesulphonamido)-ethyl]-phenoxyacetic acid or a biologically equivalent amount of another TXAA.

7. Use of PC/PCA/PG and TXAA for the manufacture of medicaments for simultaneous separate or time-lapsed administration for the treatment of thrombotic or thrombo-embolic syndromes.

8. Use of PC/PCA/PG and TXAA present in one dose unit for the manufacture of medicaments for administration in the treatment of thrombotic or thrombo-embolic syndromes.

9. Use of PC/PCA/PG and TXAA for the manufacture of medicaments according to claim 7 or 8, characterised in that the PC/PCA/PG and TXAA are in a weight ratio of from 1:1 to 1:10,000.

10. Use of 25 - 250 $\mu$g of iloprost = 5-{(E)-(1S,5S,6R,-7R)-7-hydroxy-6-[(E)-(3S,4RS)-3-hydroxy-4-methyl-1-octen-6-ynyl]-bicyclo[3.3.0]octan-3-ylidene}-pentanoic acid as the PC/PCA/PG or a biologically equivalent amount of another PC/PCA/PG for the manufacture of medicaments according to claim 7 or 8.

11. Use of 50 - 1000 mg of BM 13 177 = 4-[2-(benzenesulphonamido)-ethyl]-phenoxyacetic acid as the TXAA or a biologically equivalent amount of another TXAA for the manufacture of medicaments according to claim 7 or 8.

12. Use of PC/PCA/PG and TXAA for the manufacture of medicaments for sequential administration to avoid attenuation of the platelet aggregation inhibiting action in the treatment of thrombotic and thrombo-embolic syndromes.

## Revendications

1. Produit de combinaison à appliquer conjointement dans le cas de formes de maladies thrombotiques et thrombo-emboliques, contenant de la prostacycline, un analogue de prostacycline ou une prostaglandine (PC/PCA/PG) et un antagoniste de récepteur de thromboxane (TXAA).

2. Produit suivant la revendication 1, caractérisé en ce que PC/PCA/PG et TXAA se trouvent dans un rapport pondéral de 1/1 à 1/10.000.

3. Produit suivant la revendication 1, caractérisé en ce que PC/PCA/PG et TXAA se présentent en unités de dosage séparées.

4. Produit suivant la revendication 1, caractérisé en ce que PC/PCA/PG et TXAA se présentent ensemble dans une unité de dosage.

5. Produit suivant la revendication 1, caractérisé en ce qu'une unité de dosage de PC/PCA/PG contient 25-250 $\mu$g d'Iloprost (acide 5- {(E)-(1S,5S,6R,7R)-7-hydroxy-6-[(E)-(3S,4RS)-3-hydroxy-4-méthyl-1-octén-6-ynyl]-bicyclo[3.3.0]-octan-3-ylidène]-pentanoïque) ou une quantité biologiquement équivalente d'une autre PC/PCA/PG.

6. Produit suivant la revendication 1, caractérisé en ce qu'une unité de dosage de TXAA contient 50-1000 mg de BM 13 177 (acide 4-[2-(benzènesulfonamido)éthyl]-phénoxyacétique) ou une quantité biologiquement équivalente d'un autre TXAA.

7. Utilisation de PC/PCA/PG et de TXAA pour la préparation de médicaments à appliquer simultanément, séparément ou de manière étagée dans le temps, pour le traitement de formes de maladie thrombotiques ou thrombo-emboliques.

8. Utilisation de PC/PCA/PG et de TXAA qui se présentent en une unité de dosage, pour la préparation de médicaments à appliquer lors du traitement de formes de maladie thrombotiques ou thrombo-emboliques.

9. Utilisation de PC/PCA/PG et de TXAA pour la préparation de médicaments suivant l'une des revendications 7 et 8, caractérisée en ce que PC/PCA/PG et TXAA se trouvent dans un rapport pondéral de 1/1 à 1/10.000.

10. Utilisation de 25-250 $\mu$g d'Iloprost (acide 5-{(E)-(1S,5S,6R,7R)-7-hydroxy-6-[(E)-(3S,4RS)-3-hydroxy-4-méthyl-1-octén-6-ynyl]-bicyclo[3.3.0]-octan-3-ylidène}-pentanoïque) comme PC/PCA/PG ou d'une quantité biologiquement équivalente d'une autre PC/PCA/PG, pour la préparation des médicaments suivant l'une des revendications 7 et 8.

11. Utilisation de 50-1000 mg de BM 13 177 (acide 4-[2-( benzènesulfonamido)-éthyl]-phénoxyacétique comme TXAA ou d'une quantité biologiquement équivalente d'un autre TXAA pour la préparation de médicaments selon l'une des revendications 7 et 8.

12. Utilisation de PC/PCA/PG et de TXAA pour la préparation de médicaments à appliquer de manière séquentielle pour éviter l'affaiblissement de l'action de l'inhibition de l'agrégation des plaquettes, lors du traitement de formes de maladie thrombotiques et thrombo-emboliques.